# EUROPEAN PATENT APPLICATION

(11) **EP 4 595 994 A1**
(43) Date of publication of application: **06.08.2025**
(21) Application number: 24155194.4
(22) Date of filing: 01.02.2024
(51) Int. Cl.: A61M 1/36, A61M 5/14

(54) **METHOD FOR VENTING AN EXTRACORPOREAL CIRCUIT OF AN EXTRACORPOREAL BLOOD TREATMENT DEVICE, EXTRACORPOREAL BLOOD TREATMENT DEVICE**

(71) Applicant: B. Braun Avitum AG, 34312 Melsungen (DE)
(72) Inventor: RITTER, Kai-Uwe, Bethlehem, PA 18017 (US); SMITH, Kelly, Stroudsburg, PA 18360 (US); GUERRO, Brian, Wind Gap, PA 18091 (US)
(74) Representative: Winter, Brandl - Partnerschaft mbB

(57) **Abstract**

The invention relates to a method for priming an extracorporeal circuit (5) of an extracorporeal blood treatment device (1), the extracorporeal circuit (5) comprising a hose (50) configured to carry a priming fluid, a fluid pump (46) configured to convey the priming fluid, and a chamber (58) at least configured to vent air from the extracorporeal circuit (5), the chamber (58) comprising an inlet (64) connected to a first section (66) of the hose (50) to supply the priming fluid to the chamber (58), an outlet (68) connected to a second section (70) of the hose (50) to drain the priming fluid from the chamber (58), and a vent (72) configured to vent air from the chamber (58), wherein a stub (74) extends from the vent (72) and is sealed at its distal end by a, preferably hydrophobic, vent membrane (76) being permeable for air, the method comprising the actions of: conveying (S3) the priming fluid through the extracorporeal circuit (5), in particular by running the fluid pump (46); displacing (S4) the air from the chamber (58) into the stub (74) and through the vent membrane (76) till the priming fluid stands at the vent membrane (76); and inducing (S6) a negative pressure inside the chamber (58) such that air is sucked through the vent membrane (76) into the stub (74) and displacing the priming fluid from the stub (74) into the chamber (58). The present disclosure also relates to an extracorporeal blood treatment device (1).

## Description

### Technical Field

The present disclosure relates to a method for venting, in particular priming, an extracorporeal circuit of an extracorporeal blood treatment device according to claim 1 and an extracorporeal blood treatment device according to claim 7.

### Technical background

Extracorporeal (blood) circuits for carrying blood outside a patient's body, in particular during a blood treatment or purification procedure like dialysis, are well known. Such extracorporeal circuits are filled with a priming-fluid, -solution or -liquid, respectively, prior to the start of a treatment. This filling of the extracorporeal circuit, and in particular a dialyzer, with priming solution (e.g. NaCl) or priming fluid (priming), respectively, serves to prepare the extracorporeal circuit and the dialyzer for the subsequent treatment. In particular, priming serves to vent the extracorporeal circuit and to displace air still present in the extracorporeal circuit, in particular in the dialyzer, and in particular on the membrane of the dialyzer, prior to treatment with a physiologically compatible liquid or solution in order to prevent this air from entering the patient's vascular system during and in particular at the beginning of the blood treatment.

When the dialyzer and a hose system forming the extracorporeal (blood) circuit have been prepared, i.e. when said priming and an additional flushing (method) of the latter have been performed and terminated, the patient can basically be connected, and the priming or rinsing fluid is drained from the completely filled system and is replaced on one side by the patient's blood.

The present disclosure relates to venting, in particular to priming, i.e., preparation of the extracorporeal blood treatment device. In other words, connection/treatment of a patient is not a subject matter of the present disclosure, so that, according to the disclosure, no contact of the extracorporeal blood treatment device with a patient is provided. The present disclosure in other words relates to a method which is terminated before a patient is connected to the extracorporeal blood treatment device.

### Prior art

Prior art EP 2 953 662 B1 discloses venting from a housing of a dialyzer to remove air from the dialyzer's membrane, in particular to remove residual air from the pores of the membrane. A vacuum pump induces a negative pressure on the dialysate side of the dialysis fluid circuit. The air detached from the membrane can vent through openings of the housing covered by caps being hydrophobic and permeable for the air/ gas.

Prior art document US 2022/0133969 A1 proposes a venting on the side of the extracorporeal circuit, wherein a venting opening is disposed on the highest point of the housing of the dialyzer being covered by a membrane.

Dialysis machines usually comprise a sensor for monitoring the arterial and venous pressure. The monitoring of the arterial pressure can give indications that kinking or fistula needle issues or access issues occur. The monitoring of the venous pressure can be used to detect occlusion/kinking as well as venous needle dislodgement. The pressure is usually transferred from the fluid side (in the treatment blood side) to an air side, which can be technically realized by means of a "POD" or by a transducer protector "TP". A hose connects to a pressure sensor that converts air pressure into an electrical signal.

For removing any air, that may have been introduced into the circuit during the therapy, from the blood, before it re-enters the patient, a venous chamber may be placed in the extracorporeal circuit. The air would be collected in this chamber instead of being passed on to the patient.

During priming, the level of fluid in this chamber needs to be set to the intended level. For so called airless systems this level would be completely full, with no air left in the chamber. For other systems that level may be 2/3 of the chamber height. A prior art solution is to provide manual venting of the chamber, in particular by manually opening and closing a hose, e.g. by opening a Luer cap. In this manual configuration there is a risk the venting is not executed and the chamber cannot fill with priming fluid and the air in the chamber cannot escape. In an alternative solution the chamber can be vented automatically by the machine, if a transducer protector is used and the machine has the capability to open a valve to the environment.

The TP can fail, once the tubing to the TP fills with fluid and the TP gets wet. As there is a direct contact of the fluid to the air, in a treatment this is a blood to air contact, using the TP can cause coagulation of blood. In case of using a POD this cannot occur as the fluid is separated from the air by a flexible diaphragm. Disadvantages of the POD, however, are risk of leakage and difficulties in adjustment. Both, the TP and the POD, do not enable automatic air removal without additional measures.

Beyond the necessity to vent, in particular to prime, it is required to ensure the tightness of the extracorporeal circuit to prevent the entry of air and the negative consequences thereof. For this purpose the extracorporeal circuit may be tested before the treatment by inducing a positive pressure on the circuit.

### Brief Description of the Disclosure

Against this background, it is the task of the present disclosure to further improve a venting, in particular a priming, of an extracorporeal circuit of an extracorporeal blood treatment device and at the same time to reliably ensure the tightness of the extracorporeal circuit.

This task is solved by a method according to claim 1 and by a device according to claim 7. Advantageous embodiments of the disclosure are subject-matters of the dependent claims and/ or are described herein below.

The disclosure relates to a method for venting, in particular for priming, an extracorporeal circuit of an extracorporeal blood treatment device. The device can be a single blood line, e.g. for dosing a medicament or nutrition, or a complex medical device, e.g. a dialysis machine. The device comprises at least an extracorporeal circuit comprising a hose configured to carry a priming fluid, a fluid pump configured to convey the priming fluid, and a chamber being at least configured to vent air from the extracorporeal circuit, particularly additionally to dose a medicament or a nutrition. The chamber comprises an inlet connected to a first section of the hose to supply the priming fluid to the chamber, an outlet connected to a second section of the hose to drain the priming fluid from the chamber, and a vent configured to vent air from the chamber. A stub extends from the vent. In particular the stub is an elongated stub. The stub is sealed at its distal end, which is distal/ away from the chamber, by a, preferably hydrophobic, vent membrane which is permeable for air.

A pore size of the vent membrane preferably is chosen to prevent microbial and viral ingress, e.g. it is chosen to 0.2µm or less. The method comprises the actions of:
a) conveying the priming fluid through the extracorporeal circuit, in particular by running the fluid pump, preferably in a forward direction; thereby the priming fluid is causing
b) displacing the air from the chamber into the stub and through the (hydrophobic) vent membrane, till the priming fluid stands at the vent membrane, in particular as the (hydrophobic) vent membrane is impermeable to the fluid; and
c) inducing a negative pressure inside the chamber, such that air is sucked through the vent membrane into the stub and displacing the priming fluid from the stub into the chamber, in particular filling the stub with air again.

The action of inducing the negative pressure may be performed in at least one of the following manners: repeatedly, periodically, when required or on demand.

It is to be understood that the method of the present disclosure is terminated before a patient is connected to the extracorporeal blood treatment device, i.e. the method is no method for treatment of the human or animal body by surgery or therapy and no diagnostic method practiced on the human or animal body.

The vent membrane allows an automatic filling of the chamber with the priming fluid for a first time as it lets an air cushion, which is displaced into the stub by the priming fluid fed, pass through. When this air cushion has passed, the priming fluid stands at the vent membrane and blocks the venting membrane from inside. This blocking is advantageous for reliably testing the extracorporeal circuit as the positive pressure imposed on the extracorporeal circuit during testing would press any residual aircushion through the vent membrane, thus erroneously indicating a leakage. Due to capillary effects the fluid will not drain from the stub by itself, which is, as explained above, advantageous for performing a reliable testing. On the other hand, once blocking the vent membrane with fluid, no more venting will be possible. The action of inducing the negative pressure inside the chamber according to the disclosure frees the stub from the fluid, sucking air through the vent membrane back into the stub. Thus, the air which the priming fluid is additionally/ permanently conveying to the chamber, can enter the stub again and pass the vent membrane again, thus venting the extracorporeal circuit again. In other words, the action of inducing the negative pressure inside the chamber is to prepare for the device to allow a second or more venting of the extracorporeal circuit via the chamber and the stub if required. Preferably the action of inducing the negative pressure inside the chamber may be executed regularly, periodically, as required and/ or on demand.

Thus, by applying the method according to the disclosure, the venting, in particular the priming, of the extracorporeal circuit of the extracorporeal blood treatment device is improved and at the same time the tightness of the extracorporeal circuit can be reliably tested and ensured.

Preferably, after the action of inducing the negative pressure has ended, since a pathway through the stub is open again/ free of priming fluid, the priming, in particular the conveying, can be re-started. Thus the chamber will fill up completely with priming fluid, displacing/ purging the air which was already in the chamber plus the small amount of air pulled in from the stub through the vent membrane, until the priming fluid stands at the vent membrane. From that point on the priming can be stopped or interrupted. This method can be repeated several times until preferably all air is removed from the extracorporeal circuit.

To effectively ensure the inducing of the negative pressure, in particular to prevent a reflux via the outlet of the chamber back into the chamber, the method preferably further comprises an action of shutting off the outlet of the chamber or shutting off the second section of the hose which drains the priming fluid from the chamber. This shutting off preferably is performed by closing a shut off valve.

Advantageously the method further comprises the action of: Running the fluid pump in a reverse direction, or more generally, changing a conveying direction of the fluid pump, to induce the negative pressure according to the disclosure. The shutting off of the outlet of the chamber preferably is performed before or in coincidence with the action of running the fluid pump in a reverse direction, or changing the conveying direction of the fluid pump respectively.

Preferably the extracorporeal blood treatment device is a dialysis machine comprising a dialyzer, a dialysis fluid circuit and a dialyzer membrane separating the dialysis fluid circuit from the extracorporeal circuit inside the dialyzer.

In the case of the extracorporeal blood treatment device comprising the dialyzer, the dialysis fluid circuit and the dialyzer membrane separating the dialysis fluid circuit from the extracorporeal circuit in the dialyzer, the method advantageously further comprises the action of: stopping the fluid pump and pulling an amount of the priming fluid, in particular an amount in scale of milliliters, through the dialyzer membrane by ultrafiltration, to induce the negative pressure according to the disclosure. This shutting off of the outlet of the chamber preferably is performed before or in coincidence with the action of stopping the fluid pump and pulling the amount of the priming fluid, in particular the amount in scale of milliliters, through the dialyzer membrane by ultrafiltration. The pulling of the amount of the priming fluid, in particular the amount in scale of milliliters, through the dialyzer membrane by ultrafiltration, is preferably executed by an ultrafiltration pump of the extracorporeal blood treatment device, this ultrafiltration pump being fluidly connected to a dialysate outlet of the dialyzer.

Preferably a flow of fresh dialysis fluid to the dialyzer and a flow of used dialysate from the dialyzer is controlled to be the exactly the same. This is obtained by a balance chamber and by controlling a dialysate pump which is feeding the balance chamber with used dialysate from the dialyzer and by controlling a dialysis fluid feeding pump which is feeding the balance chamber with fresh dialysis fluid. The ultrafiltration pump is controlled to remove a specified amount from the dialysate which is feeded to the balance chamber by the dialysate pump. This results in this amount/ the milliliters of the priming fluid being drawn from the extracorporeal circuit through the dialyzer membrane into the dialysis fluid circuit which results in the inducing of the negative pressure in the chamber of the extracorporeal circuit.

In order to only induce the negative pressure when required or on demand, the method further comprises the actions of determining conditions: determining, if the priming fluid stands at the vent membrane; and, determining if the air is entering and/ or is present and/ or is accumulating in the chamber. Both conditions may be visually determined by a medical staff or automatically determined by one or more detection means, such as level sensors. And if both is determined, performing the action of inducing the negative pressure according to the disclosure.

Alternatively or additionally to performing the action of inducing the negative pressure only when it is required, it may be performed repeatedly, regularly and/ or periodically, in particular independent from the conditions.

To limit the action of inducing the negative pressure according to the disclosure to a minimum time span, the method advantageously further comprises an action of:
determining if the air has displaced, preferably fully displaced, the priming fluid from the stub into the chamber, either visually determined by a medical staff or automatically by one or more detection means, such as level sensors or air bubble sensors; and if this is determined re-starting the actions of: conveying the priming fluid through the extracorporeal circuit, in particular by opening the outlet of the chamber or the second section of the hose, in particular by opening the shut off valve, and running the fluid pump in the forward direction; and displacing the air from the chamber into the stub and through the vent membrane till the priming fluid stands at the vent membrane.

Advantageously the method further comprises a preliminary action of: connecting a container or a reservoir or a bag of the priming fluid to the extracorporeal circuit.

Advantageously the method further comprises an action of: ending the method on demand and/ or when no more air entering into and/ or being present in and/ or accumulating in the chamber is determined and/ or when the chamber and the stub are filled with the priming fluid is determined. The determination(s) may be performed visually, e.g. by a medical staff, and/ or automatically by one or more detection means, such as at least one level sensor and/ or air bubble sensor.

The disclosure further relates to an extracorporeal blood treatment device prepared for venting thereof, in particular for priming, said device comprising an extracorporeal circuit comprising a hose configured to carry a priming fluid, a fluid pump being configured to convey the priming fluid, and a chamber being at least configured to vent air from the extracorporeal circuit, preferably further being configured to dose a medicament or a nutrition. The chamber comprising an inlet connected to a first section of the hose to supply the priming fluid to the chamber, an outlet connected to a second section of the hose to drain the priming fluid from the chamber, and a vent configured to vent air from the chamber and thus venting the extracorporeal circuit. According to the disclosure a stub extends from the vent, in particular in an elongated shape. The stub may be rigid or it may be flexible to improve operability. The stub is sealed at its distal end, which means distal/ away with respect to the chamber, by a, preferably hydrophobic, vent membrane, which is permeable for air. The device is configured to induce a negative pressure inside the chamber - which means a lower pressure on the inside of the vent membrane with reference to the pressure outside of the vent membrane - such that air is sucked through the vent membrane into the stub and displaces the priming fluid from the stub into the chamber. The device may be configured to induce the negative pressure in at least one of the following manners: repeatedly, periodically, when required or on demand. A pore size of the vent membrane preferably is chosen to prevent microbial and viral ingress, e.g. it is chosen to 0.2µm or less.

Thus the venting, in particular the priming, of the extracorporeal circuit of the extracorporeal blood treatment device is improved and at the same time the tightness of the extracorporeal circuit can be reliably tested and ensured. The further advantages of the device resulting from stub and the inducing of the negative pressure according to the disclosure have already been described in the preceding description. In order to not overload this document, the advantages will not be repeated.

In a preferred embodiment the stub is elongated so that after the venting, in particular priming of the extracorporeal circuit an elongated volume of priming fluid is filling the stub and standing at the vent membrane. This elongated volume is held in the stub by capillary force/ effect. Thus, in an extracorporeal blood treatment device according to the present disclosure, the vent membrane is securely separated from blood which passes through the chamber, so that coagulation is excluded in the region of the vent and the stub.

It is advantageous if a control (unit) is provided, the control (unit) being configured to control the fluid pump to run in a reverse direction while or after the outlet of the chamber or the second section of the hose are controlled to be shut off, in particular shut off by closing a shut-off valve of the extracorporeal circuit, thereby inducing the negative pressure.

Further it is advantageous if the device comprises a dialyzer, a dialysis fluid circuit with a dialysate pump and a dialyzer membrane separating the dialysis fluid circuit from the extracorporeal circuit in the dialyzer, and that the control unit is configured to control the fluid pump to stop while or after the outlet of the chamber or the second section of the hose is shut off, in particular shut off by closing a shut-off valve of the extracorporeal circuit, such that a fluid volume is occluded in the extracorporeal circuit, and to control the dialysate pump or an ultrafiltration pump of the extracorporeal blood treatment device to pull an amount of the fluid, in particular an amount in scale of milliliters, from the extracorporeal circuit through the dialyzer membrane to the dialysis fluid circuit by ultrafiltration, thereby inducing the negative pressure.

To enable the priming fluid to rest in the stub and not be drained from the stub by gravity, it is advantageous that the stub is configured to have a capillary effect with respect to the priming fluid applied. In particular an inner diameter of the stub may be suitably chosen. Preferably the inner diameter of the stub is 2 mm to 6 mm.

To act as a sterile barrier with respect to the environment it is advantageous when a pore diameter of the vent membrane is preferably 0.2 µm or less. Thus, apart from the vent membrane no supplementary cap is required at the distal end of the stub.

In order to secure the hydrophobic function it is advantageous if the vent membrane is configured to be wetted multiple times, in particular if the material of the vent membrane is adequately selected, e.g. such as PTFE.

Preferably in the extracorporeal circuit a pressure sensor unit is provided, being configured to measure a pressure of the priming fluid in the hose, comprising a flexible diaphragm separating the fluid from an air cushion, the pressure sensor unit converting an air-pressure of the air cushion into an electrical signal, and preferably transferring the signal to the control unit, which is enabled to execute a pressure based tightness or leakage testing of the extracorporeal circuit. Such type of a pressure sensor unit is known as a "POD", which securely separates the blood side from the air cushion by the flexible diaphragm, so that in this area of the POD coagulation is excluded. This is particularly advantageous in view of the negative pressure which is induced according to the disclosure which could lead to a penetration of air into the venous hose if a simple transducer protector without such a blood-air separating diaphragm was used instead of a POD.

Advantageously the device, in particular its control unit, is configured to control a leaking test on the extracorporeal circuit, by controlling the fluid pump to induce a predetermined positive testing pressure on the extracorporeal circuit, in particular by controlling a forward direction of the fluid pump, and by controlling the shut-off valve downstream the chamber and a shut-off valve upstream the chamber to shut off for a predetermined time period, and to evaluate a pressure drop detected by the pressure sensor unit. Thus the integrity of the extracorporeal circuit, in particular of the vent membrane, can be tested.

In order to be able to perform this testing safely with respect to the environment and to obtain a reliable result it is necessary that a water breakthrough pressure of the vent membrane is higher than the predetermined positive testing pressure and/ or higher than high pressure that can occur.

Advantageously the vent is located at a highest point of the chamber, thus the stub extends from the highest point of the chamber, in particular the highest point with respect to gravity. Alternatively or additionally a wall of the chamber leading to the vent, which means leading to the stub, is sloped or is dome-shaped, thereby ensuring that the air reliably can flow to the stub due to its lower density.

Further, advantageously the inlet of the chamber is located at a high point and/ or it extends with a tube into the inner volume of the chamber, thus the risk of foam in the chamber due to dripping priming fluid is reduced.

A clamp may be provided on the stub, to enable medication/ introducing medication during a treatment through the stub. This clamp has to be closed when the medication is to be connected, in order to avoid priming fluid (or blood during the treatment) leaving the circuit. After the connection of the medication, the clamp is preferably opened again.

Else, if no medication is performed, said clamp is not required on the stub, which is an advantage, as the vent membrane is sealing the vent/ the stub safely, as described above.

Advantageously the vent membrane is part of a membrane device, in particular a filter device, detachably mounted on the distal end of the stub, to enable an exchange of the vent membrane and/ or to enable a connection of a medication or a nutrition to the extracorporeal circuit.

Advantageously the membrane device has one Luer connector on one side or one Luer connector on both sides of the vent membrane, for easy connection to the distal end of the stub.

In an advantageous manner the extracorporeal blood treatment device according to at least one aspect of the preceding description, in particular its control unit, is configured to store and/ or to carry out and / or to apply the method according to the disclosure.

In the following, an embodiment of the present disclosure is described in detail with reference to the accompanying drawings.

### Brief Description of the Figures

Fig. 1 schematically shows an extracorporeal blood treatment device configured as a dialysis machine during an active use with a patient who is connected to the machine;
Fig. 2 schematically shows an extracorporeal circuit to be vented or primed of the dialysis machine according to fig. 1 according to a preferred embodiment;
Figs. 3a to 3h schematically show a chamber of the extracorporeal circuit according to figure 1, configured to vent the extracorporeal circuit, each figure showing a different state of the chamber, corresponding with actions of a preferred embodiment of a method according to fig. 4; and
Fig. 4 shows a method for venting or priming the extracorporeal circuit according figs. 1 and 2, actions of the method corresponding to the different states of the chamber according to fig. 3.

### Summary

In summary, according to an embodiment of the disclosure, a method for venting air or gas from an extracorporeal circuit of an extracorporeal blood treatment device, and an extracorporeal blood treatment device, such as a dialysis machine, comprising such an extracorporeal circuit to be vented, will be described.

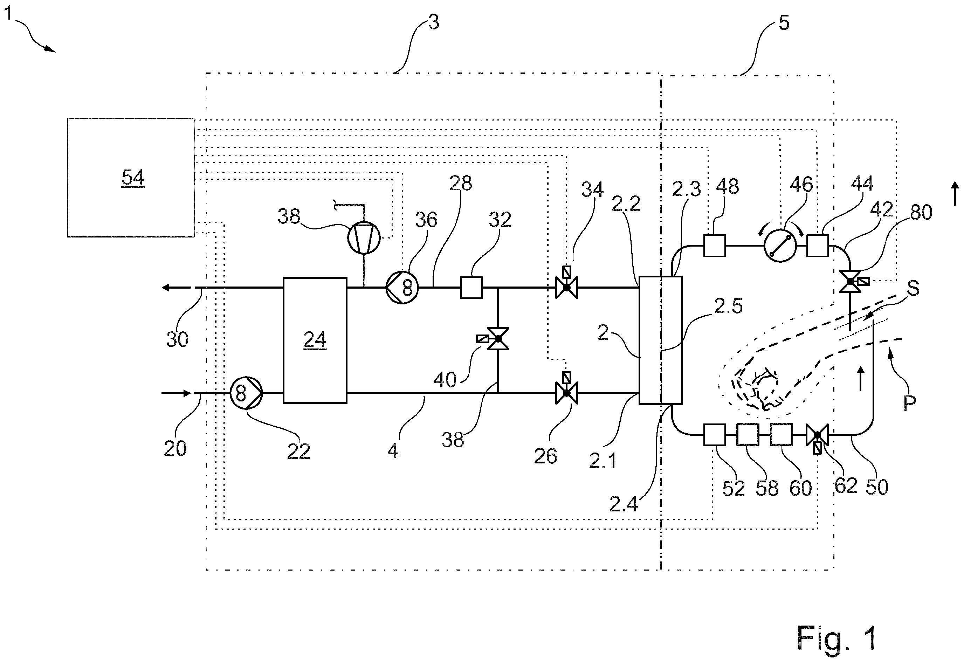

Figure 1 shows a schematic view of an extracorporeal blood treatment device 1 in the form of a dialysis machine for extracorporeal treatment of blood of a patient P. The following description relating to fig. 1 describes the extracorporeal blood treatment device 1 and the extracorporeal treatment of blood of the patient for information. It is added that the extracorporeal treatment of blood of the patient is not comprised in the method of the present disclosure, since the method is terminated before the patient is connected to the extracorporeal blood treatment circuit. The dialysis machine 1 comprises as a main component a dialyzer 2 with a dialysis fluid inlet 2.1 and a dialysate outlet 2.2 and a fluid inlet or blood inlet 2.3 (in the following only fluid inlet 2.3) and a fluid outlet or blood outlet 2.4. (in the following only fluid outlet 2.4). Internally, the dialyzer 2 is separated into a dialysis fluid side and a fluid side or blood side (in the following only fluid side) by means of a semipermeable membrane 2.5.

The dialysis fluid inlet 2.1 is fluidly connected to a dialysis fluid supply 20 via a dialysis fluid feed path 4 via which fresh and individually prepared dialysis fluid is feeded to the dialysis fluid inlet 2.1. In the dialysis fluid feed path 4 a dialysis fluid feeding pump 22 is provided to convey the dialysis fluid via a balancing chamber 24 and a shut off valve 26 to the dialysis fluid inlet 2.1. The shut off valve 26 is provided to shut off the dialysis fluid inlet 2.1 and can be controlled by a control unit 54 of the dialysis machine 1, the shut off valve 26 being signally connected to the control unit 54.

The dialysate outlet 2.2 is fluidly connected to a dialysate drain 30 via a dialysate drain path 28 along which is provided a shut off valve 34 for shutting off the dialysate outlet 2.2, being preferably of the same configuration as the shut off valve 26, and also being signally connected to the control unit 54. The drain path 28 further comprises a concentration determining unit 32 to determine a concentration of a component in the dialysate and a dialysate pump 36 to convey the dialysate, or in particular to apply a negative pressure to the dialysate outlet 2.2, if required. The dialysate pump 36 is connected to the dialysate drain via the balancing chamber 24 which is configured to ensure that a desired volume of excess water can be removed from the patient's blood during ultrafiltration.

Downstream the dialysate pump 36 and upstream the balance chamber 24 an ultrafiltration flow path branches off from the drain path 28. The ultrafiltration flow path has an ultrafiltration pump 38 with an inlet being in fluid connection with the drain flow path 28. An outlet of the ultrafiltration pump 38 is in fluid connection (not shown) with the dialysate drain 30. According to the disclosure the ultrafiltration pump 38 may draw a specific amount of dialysate from the drain flow path 28 to induce a negative pressure in an extracorporeal circuit 5 (to be explained below).

The extracorporeal circuit 5 of the dialysis machine 1 comprises an arterial hose section 42 to take blood from the Patient P and to deliver it to the fluid inlet 2.3 of the dialyzer 2. Along the arterial hose section 42 an arterial shut off valve 80, an arterial pressure sensor 44, a fluid pump 46 and a blood entry pressure sensor 48 are fluidly connected. The fluid pump 46 is configured to be operable in both directions which means to generate a fluid flow in two opposite directions, e.g. in a forward direction F to deliver or convey fluid or blood to the fluid inlet 2.3, and in an opposite or reverse direction R, e.g. to induce a negative pressure on a volume being occluded in the extracorporeal circuit 5 (to be explained below). A further flow path of the extracorporeal circuit 5 extends through the arterial hose section 42 to the fluid inlet 2.3 and through an extracorporeal side of the membrane 2.5 to the fluid outlet 2.4, being connected to a venous hose section 50 leading back to the shunt S. Along said venous hose section 50 a fluid outlet pressure sensor 52, designed as a POD, a venting chamber 58 configured to remove air or gas from the extracorporeal circuit 5, an air bubble detector 60 configured to detect and signal towards the control unit 54 the presence of air downstream from chamber 58, and a venous shut off valve 62 are provided. To filter coagulated blood a coagulation filter may be provided, e.g. downstream of the chamber outlet 68.

Pressure measuring is in particular important for testing the integrity of the extracorporeal circuit 5. A POD type pressure transducer, in contrast to a transducer protector TP, advantageously avoids fluid air contact.

In the following a method relating to a preferred embodiment of the disclosure for venting or priming the extracorporeal circuit 5 of the extracorporeal blood treatment device 1 according to figure 1 as well as these aspects of the extracorporeal blood treatment device 1 relating to the preferred embodiment, are disclosed, referring to figures 1, 2, 3a to 3h and 4.

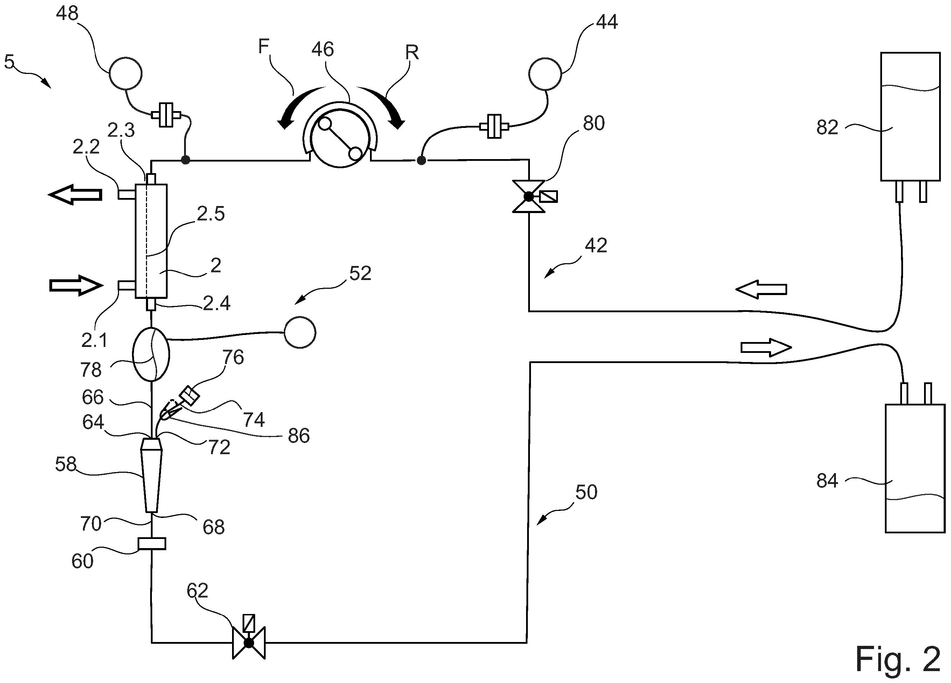

Figure 2 shows the extracorporeal circuit 5 of the dialysis machine 1 according to figure 1 in a more detailed manner. The extracorporeal circuit 5 according to figure 2 is prepared to be vented or primed previous to an extracorporeal blood treatment of a/ the patient P to be performed afterwards. In other words, the extracorporeal circuit 5 is not connected to a/ the patient P and all actions of the method according to the disclosure are executed independently from a/ the patient P.

For venting/ priming the extracorporeal circuit 5 a priming bag or saline bag 82 containing venting/ priming solution (in the following just named fluid) is fluidly connected to the arterial hose section 42 of the extracorporeal circuit 5. The fluid is filled from the bag 82 into the extracorporeal circuit 5. In order to ensure that the dialyzer 2 is properly filled with fluid, the dialyzer 2 can be rotated in between in order to mobilize and remove remaining air bubbles, wherein the rotating can be repeatedly executed due to a usual dialyzer membrane 2.5 having about 1.5 m² of membrane surface and being hydrophobic. Thus it is very probable that air bubbles remove better by imposing a mechanical or pressure impulse to the dialyzer 2/ the fluid. A first amount of the fluid which has passed the arterial hose section 42, the dialyzer 2 and the venous hose section 50 is collected in a drain bag 84 connected to the venous hose section 50.

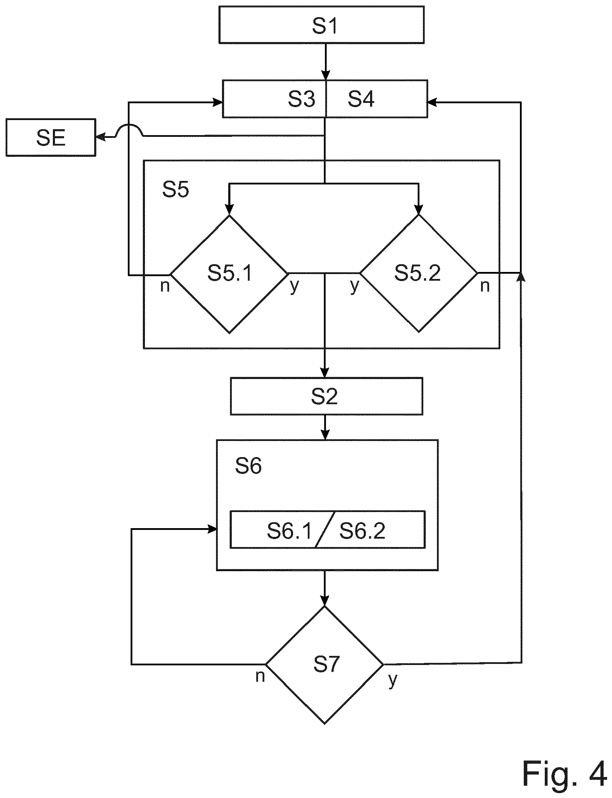

These actions, from connecting the saline bag 82 to the arterial hose section 42 up to collecting the first amount of the fluid in the empty bag 84, are summarized as an action of: "Starting S1" the venting or priming of the extracorporeal circuit 5 of the extracorporeal blood treatment device 1, according to the method of the present disclosure (see fig. 4).

According to figure 2, instead of directly transferring the pressure from the fluid of the venous hose section 50 to an air cushion, the POD type fluid outlet pressure sensor unit 52 separates fluid from air by a flexible diaphragm 78. The fluid side of the diaphragm 78 is fluidly connected to the venous hose section 50, the air cushion side is pneumatically connected to the pressure sensor of the fluid outlet pressure sensor unit 52. Due to a change in pressure within the venous hose section 50, the diaphragm 78 is deflected, and this force effect is transmitted via the air cushion to said sensor, which is measuring the internal pressure of the venous hose section 50.

For the purpose of the method, after the action of "Starting S1" the venting or priming of the extracorporeal circuit 5 of the extracorporeal blood treatment device 1 is over, the following actions are carried out in accordance with the disclosure:
It is assumed, that the venous shut off valve 62 is controlled to be opened by the control unit 54 via the signal path (see fig. 1). Said control unit 54 is controlling the fluid pump 46 to run in a forward direction F and thus conveying fluid from the saline bag 82 through the dialyzer 2, the chamber 58, passing the opened, venous shut off valve 62 and being ducted to the empty bag 84. In terms of the method according to the disclosure, this action is: "Conveying S3" the priming fluid through the extracorporeal circuit 5, in particular by running the fluid pump 46, in particular in a forward direction (see fig. 4).

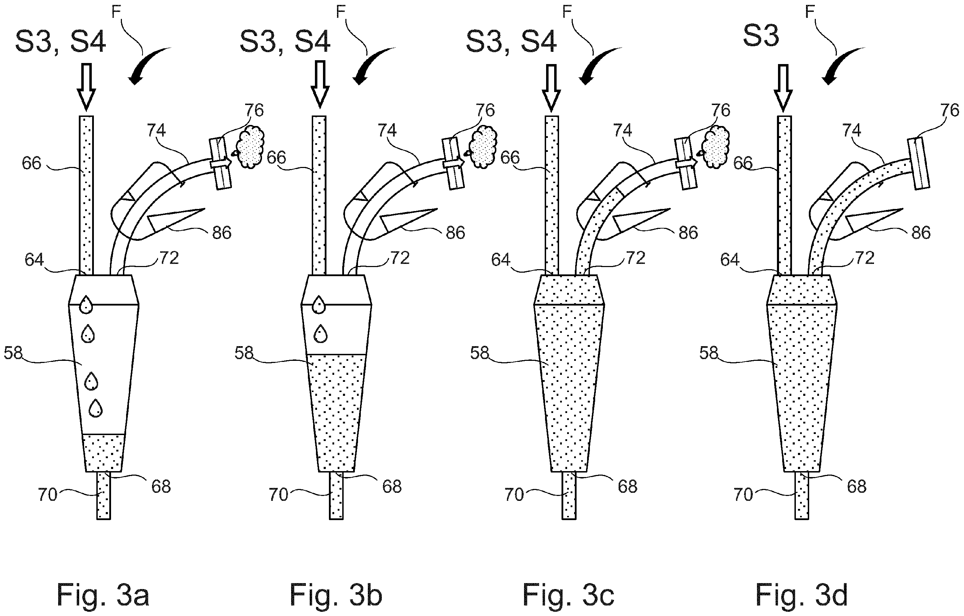

Figure 3a presents the effect which the action of "Conveying S3" has on the chamber 58. Through the fluid inlet 64 of the chamber 58 the fluid, having passed the extracorporeal side of the dialyzer 2 and draining air from the whole fluid path, is entering/ dripping into the chamber 58. Inside the chamber 58 fluid and air are naturally separated due to different densities, so that the air is collected in the upper part of the chamber 58, where the vent (opening) 72, and the stub 74 extending thereof, are located. The stub 74 is (still) empty, which means filled with air. As mentioned, the stub 74 is sealed by the vent membrane 76. The vent membrane 76 in this embodiment is part of a filter device, having Luer connectors on each side of the vent membrane 76, which enables the medical staff to quickly and safely detach and reattach the filter device from and to the distal end section of the stub 74, e.g. for the purpose of a medication throughout the blood treatment.

Given that a flow resistance downstream the chamber 58 is sufficiently high, inside the chamber 58 the fluid starts to accumulate and rise in level. In other words, the action of "Conveying S3" entails a simultaneous action of: "Displacing S4" the air from the chamber 58 into the stub 74 and through the vent membrane 76 till the priming fluid stands at the vent membrane 76 (see fig. 4).

The effect of simultaneous actions "Conveying S3" and "Displacing S4" are well visualized in figures 3a to 3d, wherein a level of the fluid is rising up inside the chamber 58 in figures 3a, 3b, rising up the stub 74 in figure 3c and finally standing at the vent membrane 76 in figure 3d. As the vent membrane 76 is permeable to gas it lets the air being displaced by the rising fluid pass (see fig. 3a to 3c), until the fluid stands at the vent membrane 76 (see fig. 3d). Then venting/ priming by the vent 72 and the vent membrane 76 stops. This is a status of the venting or priming method where the extracorporeal circuit 5 has been successfully vented/ primed and potentially all air has been removed. This could be a point in time where the blood treatment could be started.

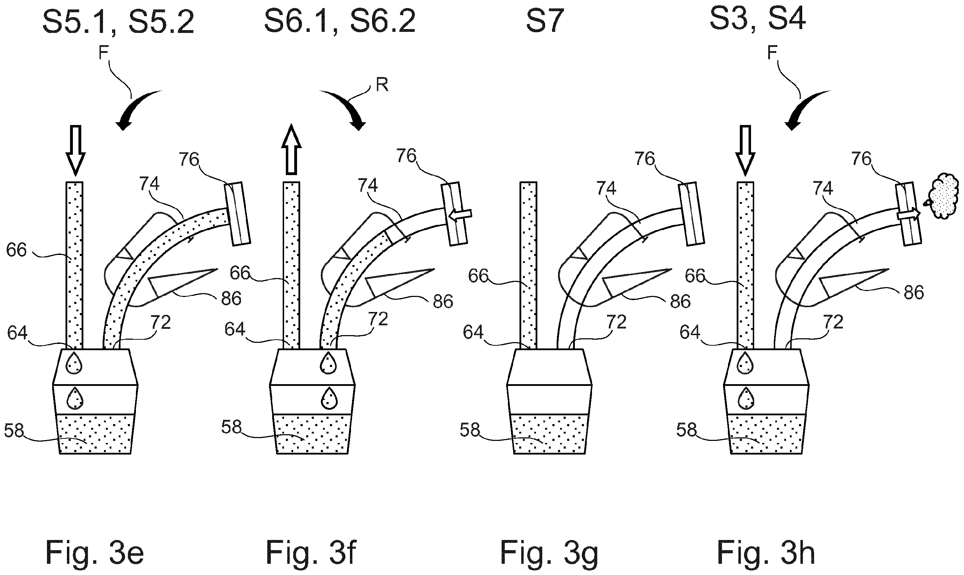

In reality however, it may occur that not all of the air of the extracorporeal circuit 5 has already been removed at that point and that residual air cushions or bubbles not yet being mobilized may get activated. This would lead to a situation where the chamber 58 is still feeded with fluid through fluid inlet 64, said fluid flow is still - or again - carrying air into the chamber 58, which is illustrated in figure 3e.

As the stub 74 is designed by purpose to have capillary effect (which will be explained below), the fluid will not drain from the stub 74 by gravity force alone. So to reopen the stub 74, or in other words to remove the fluid from the stub 74 to make a passage of air through the vent membrane 76 possible again, the method further comprises the action, according to the disclosure, of: "Inducing S6" a negative pressure inside the chamber 58 such that air is sucked through the vent membrane 76 into the stub 74 and displacing the priming fluid from the stub 74 into the chamber 58 (see fig. 3f).

In the embodiment of the method according to figure 4 the action of inducing S6 the negative pressure on the chamber 58 is exclusively executed on demand, which means if the aforementioned preconditions - the stub 74 is filled with fluid which stands at the vent membrane 76 and there is air in the chamber 58 - are actually met. To check these preconditions the method may comprise the two actions of: "Determining S5.1" if the priming fluid stands at the vent membrane 76; and "Determining S5.2" if the air is entering and/ or is present and/ or is accumulating in the chamber 58. If both preconditions are met the action of "Inducing S6" the negative pressure on the chamber 58 is performed (see fig. 4). If one is not met, the actions of "Conveying S3" and "Displacing S4" may be continued.

Alternatively or in addition to this when-required-like or on-demand-like action of "Inducing S6", the action of "Inducing S6" may be executed manually, automatically, manually-repeatedly, automatically-repeatedly, manually-periodically or automatically-periodically.

Preferably, the action of "Inducing S6" the negative pressure on the chamber 58 is controlled by the control unit 54.

According to the present disclosure there are at least two advantageous (sub) actions (see fig. 4, S6.1 / S6.2) which define in which way the action of "Inducing S6" the negative pressure on the chamber 58 is performed.

A preliminary condition for both of them is that the outlet 68 of the chamber 58 or the second section 70 of the hose 50 is shut off. To obtain this it is preferred that the venous shut off valve 62 is controlled to be closed/ shut off, which is the action of: "Shutting off S2" the outlet 68 of the chamber 58 or the second section 70 of the hose 50, in particular by closing the venous shut off valve 62 (see fig. 4).

From that point the negative pressure in the chamber 58 can be induced either in a rather direct manner by controlling the fluid pump 46 from its forward direction F to its reverse direction R (see fig. 2), thus reversing the flow direction of the fluid and inducing the negative pressure in the chamber 58. For this option the method further comprises the action of: "Running S6.1" the fluid pump 46 in a reverse direction R.

On the other hand it is possible to induce the negative pressure in a more indirect manner via the dialysis fluid circuit 3. This in fact requires that the extracorporeal blood treatment device 1 comprises a dialyzer 2, the dialysis fluid circuit 3 and the dialyzer membrane 2.5 separating the dialysis fluid circuit 3 from the extracorporeal circuit 5 in the dialyzer 2. The action of "Inducing S6" the negative pressure in the chamber then can be achieved by the action of: "Stopping S6.2" the fluid pump 46 and pulling an amount of the fluid, in particular an amount in scale of milliliters, through the dialyzer membrane 2.5 by ultrafiltration, in particular by controlling the ultrafiltration pump 38 accordingly.

In this context with respect to fig. 1 the flow of the fresh dialysis fluid to the inlet 2.1 of the dialyzer 2 and the flow of the used dialysate from the outlet 2.2 of the dialyzer 2 is controlled to be exactly the same by the balance chamber 24 of the extracorporeal blood treatment device 1 and by controlling the fluid feeding pump 22 and the dialysate pump 36 accordingly. According to the disclosure the control unit 54 is controlling the fluid pump 46 to stop and the venous shut off valve 62 to close, so that a volume of fluid is occluded between the fluid pump 46 and the shut off valve 62 inside the extracorporeal circuit 5. In addition the ultrafiltration pump 38 is controlled to remove a specified amount of dialysate from the dialysis fluid circuit 3, which means from the dialysate drain path 28, so that less dialysate is entering the balance chamber 24. This results in the pulling of the amount/ the milliliters of the priming fluid from the extracorporeal circuit 5 through the dialyzer membrane 2.5 into the dialysis fluid circuit 3 which finally results in the inducing of the negative pressure in the chamber 58 of the extracorporeal circuit 5.

To remember: the action of inducing S6 the negative pressure in the chamber 58 has the goal to empty the stub 74 from fluid so that air again can be displaced through the stub 74 to pass the vent membrane 76 to the environment and thereby removing the air from the extracorporeal circuit 5. In other words: for venting or priming it is required, that the vent membrane 76 is sufficiently dry on its inner side facing towards the chamber 58 and that no fluid stands at the vent membrane 76. In again other words, this is one condition to be met to terminate the action of "Inducing S6" the negative pressure to the chamber 58 and the method according to the disclosure further comprises the action of: "Determining S7" if the air has displaced, preferably fully displaced, the priming fluid from the stub 74 into the chamber 58. If the requirement is met/ determined, the method will restart with the actions of "Conveying S3" the priming fluid through the extracorporeal circuit 5, in particular by running the fluid pump 46 in the forward direction F and of "Displacing S4" the air from the chamber 58 into the stub 74 and through the vent membrane 76 till the priming fluid stands at the vent membrane 76.

After the venting or priming is finished, the chamber 58 and the stub 74 are filled with the priming fluid up to the vent membrane 76.

In this vented/ primed state a leakage test or integrity test can be performed on the extracorporeal circuit 5 to check if it is tight. This is required because any leak could draw air to the extracorporeal circuit 5 which is a potential danger for the patient during the planned blood treatment.

As this test is based on setting the extracorporeal circuit 5 under a positive pressure, it is first required that the extracorporeal circuit 5 does not contain air any more, and secondly, that the fluid stands at the inner side of the vent membrane 76. The first, because any air would compress under the positive pressure, thus falsifying the test result, the second, because any air standing at the inner side of the vent membrane 76 could flatten a pressure ramp to reach a predetermined positive test pressure. In other words the predetermined positive test pressure would be reached with a lower gradient or later than expected, respectively. This could be misinterpreted - either by the medical staff performing the test or by the control unit 54 having stored the predetermined positive test pressure and a predetermined ramp thereof - to be a leakage of the extracorporeal circuit 5 which might lead to a termination of the test.

Testing has revealed though, that even if there is some air in the chamber 58, the stub 74 will not drain (due to capillary as explained above) and the test can be performed successfully.

The second condition - fluid stands at the inner side of the vent membrane 76 - does not need to be met if a clamp 86 is provided at the stub 74 (see fig. 2, figs. 3). The clamp 86 can separate the fluid in the stub 74 from the air in the stub 74, or in general close the stub 74 at any position. The advantage of using the vent membrane 76 and meeting the condition that the fluid stands at the inner side of the vent membrane 76 is, that no clamp is required at all and as consequence no manual interaction is required. Such a required interaction is a disadvantage as it can be forgotten erroneously.

The predetermined positive test pressure can be achieved by the actions of:
a) Meeting requirement: no air in the chamber 58 and fluid stands at the vent membrane 76; optionally: Closing clamp 86 manually, if available;
b) Shutting off the venous shut off valve 62, the dialysis fluid shut off valve 26 and the dialysate shut off valve 34;
c) Running the fluid pump 46 in the forward direction F until the venous pressure sensor unit 52 signals the predetermined positive (test) pressure to the control unit 54;
d) Stopping the fluid pump 46 and shutting the arterial shut off valve 80;
e) tracking the pressure and/ or pressure gradient with the venous pressure sensor unit 52 for a predetermined time span, e.g. 10 to 20 seconds, preferably 15 seconds;
f) evaluating the pressure drop after the predetermined time span and providing a result about the extracorporeal circuit being tight or not.

Alternatively the positive test pressure can be induced by running the ultrafiltration pump 38.

According to figure 4 the method of venting or priming may be stopped at any time by an action of: "Stopping SE" of the venting or priming (see fig. 4). Only to not overload the diagram, according to figure 4 the action of stopping SE is just branched out from the actions S3/S4 (conveying/ displacing, see above). Of course this action "Stopping SE" may be executed at any time, during any of the actions of the method according to the present disclosure.

### List of reference signs

- 1: extracorporeal blood treatment device
- 2: dialyzer
- 2.1: dialysis fluid inlet
- 2.2: dialysis fluid outlet
- 2.3: blood (fluid) inlet
- 2.4: blood (fluid) outlet
- 2.5: dialyzer membrane
- 3: dialysis fluid circuit
- 4: dialysis fluid feed path
- 5: extracorporeal circuit
- 20: dialysis fluid supply
- 22: dialysis fluid feeding pump
- 24: balancing chamber
- 26: dialysis fluid shut off valve
- 28: dialysate drain path
- 30: dialysate drain
- 32: concentration determining unit
- 34: dialysate shut off valve
- 36: dialysate pump
- 38: ultrafiltration pump
- 42: arterial hose section
- 44: arterial pressure sensor
- 46: fluid pump
- 48: blood entry pressure sensor
- 50: venous hose section
- 52: fluid outlet pressure sensor unit
- 54: control unit
- 58: chamber
- 60: air bubble detector
- 62: venous shut off valve
- 64: fluid inlet
- 66: first section
- 68: fluid outlet
- 70: second section
- 72: vent
- 74: stub
- 76: vent membrane
- 78: flexible diaphragm
- 80: arterial shut off valve
- 82: saline bag
- 84: drain bag
- 86: stub clamp

- P: patient
- S: shunt
- S1: action of starting to vent
- S2: action of shutting off valve
- S3: action of conveying fluid
- S4: action of displacing air
- S5: action of determining conditions
- S5.1: action of determining air in chamber
- S5.2: action of determining fluid in stub
- S6: action of inducing negative pressure
- S6.1: action of reversing fluid pump
- S6.2: action of running ultrafiltration pump
- S7: action of determining air in stub
- SE: action of terminating to vent

## Claims

1. A method for venting, in particular priming, an extracorporeal circuit (5) of an extracorporeal blood treatment device (1), the extracorporeal circuit (5) comprising a hose (50) configured to carry a priming fluid, a fluid pump (46) configured to convey the priming fluid, and a chamber (58) being at least configured to vent air from the extracorporeal circuit (5), the chamber (58) comprising an inlet (64) connected to a first section (66) of the hose (50) to supply the priming fluid to the chamber (58), an outlet (68) connected to a second section (70) of the hose (50) to drain the priming fluid from the chamber (58), and a vent (72) configured to vent air from the chamber (58), wherein a, in particular elongated, stub (74) extends from the vent (72) and is sealed at its distal end by a, preferably hydrophobic, vent membrane (76) being permeable for air, the method comprising the actions of:
conveying (S3) the priming fluid through the extracorporeal circuit (5), in particular by running the fluid pump (46);
displacing (S4) the air from the chamber (58) into the stub (74) and through the vent membrane (76) till the priming fluid stands at the vent membrane (76); and
inducing (S6) a negative pressure inside the chamber (58) such that air is sucked through the vent membrane (76) into the stub (74) and displacing the priming fluid from the stub (74) into the chamber (58).

2. The method according to claim 1, comprising an action of:
shutting off (S2) the outlet (68) of the chamber (58) or the second section (70) of the hose (50), in particular by closing a shut off valve (62), preliminary to or in coincidence with the action of inducing (S6) the negative pressure inside the chamber (58).

3. The method according to claim 1 or 2, wherein the action of inducing (S6) the negative pressure comprises an action of:
running (S6.1) the fluid pump (46) in a reverse direction (R).

4. The method according to any of the proceeding claims, wherein the extracorporeal blood treatment device (1) comprises a dialyzer (2), a dialysis fluid circuit (3) and a dialyzer membrane (2.5) separating the dialysis fluid circuit (3) from the extracorporeal circuit (5) in the dialyzer (2), wherein the action of inducing (S6) the negative pressure comprises an action of:
stopping (S6.2) the fluid pump (46) and pulling an amount of the fluid, in particular an amount in scale of milliliters, through the dialyzer (2.5) membrane by ultrafiltration, in particular by an ultrafiltration pump (38) of the extracorporeal blood treatment device (1).

5. The method according to any of the proceeding claims, further comprising an action of:
determining (S5.1) if the priming fluid stands at the vent membrane (76); and
determining (S5.2) if the air is entering and/ or is present and/ or is accumulating in the chamber (58); and if both is determined, performing the action of inducing (S6) the negative pressure.

6. The method according to claim 5, further comprising an action of:
determining (S7) if the air has displaced the priming fluid from the stub (74) into the chamber (58); and if this is determined, restarting the actions of conveying (S3) the priming fluid through the extracorporeal circuit (5), in particular by opening the outlet (68) of the chamber (58) or the second section (70) of the hose (50), in particular by opening the shut off valve (62), and running the fluid pump (46) in a forward direction (F), and of displacing (S4) the air from the chamber (58) into the stub (74) and through the vent membrane (76) till the priming fluid stands at the vent membrane (76).

7. An extracorporeal blood treatment device (1) prepared for venting thereof, in particular for priming, comprising:
an extracorporeal circuit (5) comprising a hose (50), configured to carry a priming fluid,
a fluid pump (46) being configured to convey the priming fluid, and a chamber (58), being at least configured to vent air from the extracorporeal circuit (5),
the chamber (58) comprising an inlet (64) connected to a first section (66) of the hose (50) to supply the priming fluid to the chamber (58), an outlet (68) connected to a second section (70) of the hose (50) to drain the priming fluid from the chamber (58), and a vent (72), configured to vent air from the chamber (58),
**characterized in that**
a, in particular an elongated, stub (74) extends from the vent (72) and is sealed at its distal end by a, preferably hydrophobic, vent membrane (76) being permeable for air, and that the device (1) is configured to induce a negative pressure inside the chamber (58), such that air is sucked through the vent membrane (76) into the stub (74), thereby displacing the priming fluid from the stub (74) into the chamber (58).

8. The device (1) according to claim 7, **characterized in that** a control unit (54) is provided, the control unit (54) being configured to control the fluid pump (46) to run in a reverse direction (R) while or after the outlet (68) of the chamber (58) or the second section (70) of the hose (50) is shut off, in particular shut off by closing a shut-off valve (62), thereby inducing the negative pressure.

9. The device (1) according to claim 7, **characterized in that** the device comprises a dialyzer (2), a dialysis fluid circuit (3) with a dialysate pump (36) and a dialyzer membrane (2.5) separating the dialysis fluid circuit (3) from the extracorporeal circuit (5) in the dialyzer (2), and that the control unit (54) is configured to control the fluid pump (46) to stop while or after the outlet (68) of the chamber (58) or the second section (70) of the hose (50) is shut off, in particular shut off by closing a shut-off valve (62), such that a fluid volume is occluded in the extracorporeal circuit (5), and to control the dialysate pump (36) or an ultrafiltration pump (38) of the device (1) to pull an amount of the fluid, in particular an amount in scale of milliliters, from the extracorporeal circuit (5) through the dialyzer membrane (2.5) to the dialysis fluid circuit (3) by ultrafiltration, thereby inducing the negative pressure.

10. The device (1) according to one of the claims 7 to 9, **characterized in that** the stub (74), in particular an inner diameter of the stub (75), is configured to have a capillary effect with respect to the fluid.

11. The device (1) according to one of the claims 7 to 10, **characterized in that** a pore diameter of the vent membrane (76) is preferably 0.2 µm or less, to act as a sterile barrier with respect to the environment.

12. The device (1) according to one of the claims 7 to 11, **characterized in that** the vent membrane (76) is configured to be wetted multiple times, in particular the vent membrane (76) is made of a material, such as PTFE, suitable to be wetted multiple times.

13. The device (1) according to one of the claims 7 to 12, **characterized in that** the extracorporeal circuit (5) has a pressure sensor unit (52) being configured to measure a pressure of the fluid, the pressure sensor unit (52) comprising a flexible diaphragm (78) separating the fluid from an air cushion, the pressure sensor unit (52) converting an air-pressure of the air cushion into an electrical signal.

14. The device (1) according to claim 13, **characterized in that** the control unit (54) is configured to control a leaking test on the extracorporeal circuit (5) by controlling the fluid pump (46) to induce a predetermined positive testing pressure on the extracorporeal circuit (5) and by controlling the shut-off valve (62) downstream the chamber (58) and a shut-off valve (80) upstream the chamber (58) to shut off for a predetermined time period, and to evaluate a pressure drop detected by the pressure sensor unit (52).

15. The device according to claim 14, **characterized in that** a water breakthrough pressure of the vent membrane (76) is higher than the predetermined positive testing pressure, and/ or higher than other high pressures that can occur.
